# EUROPEAN PATENT SPECIFICATION

(11) **EP 4 374 829 B1**
(45) Date of publication and mention of the grant of the patent: **09.04.2025**
(21) Application number: 22209214.0
(22) Date of filing: 23.11.2022
(51) Int. Cl.: A61F 2/28, A61F 2/30

(54) **ASSEMBLEABLE ARTIFICIAL BONE PLATE AND ARTIFICIAL BONE PLATE UNIT**
ZUSAMMENSETZBARE KÜNSTLICHE KNOCHENPLATTE UND KÜNSTLICHE KNOCHENPLATTENEINHEIT
PLAQUE OSSEUSE ARTIFICIELLE ASSEMBLABLE ET UNITÉ DE PLAQUE OSSEUSE ARTIFICIELLE

(43) Date of publication of application: 29.05.2024
(73) Proprietor: Ji Yan Biomedical Co., Ltd, Taipei City (TW)
(72) Inventor: TSAI, Tung-Kuo, New Taipei City (TW); OU, Keng-Liang, Kaohsiung City (TW); SHEN, Yung-Kang, New Taipei City (TW); HUANG, Yin-Chung, New Taipei City (TW); HUNG, Kuo-Sheng, New Taipei City (TW); OU, Yu-Sin, Kaohsiung City (TW)
(74) Representative: Casalonga

(56) References cited:
- US-A1- 2019 380 836
- US-A1- 2020 405 353
- US-A1- 2022 354 553
- US-A9- 2014 195 004

## Description

### 1. Field of the Invention

The present invention is defined in claim 1 and relates to a medical implant, especially to an assembleable artificial bone plate and an artificial bone plate unit that can be used for cranioplasty, i.e., surgical repair of a bone defect in the skull.

### 2. Description of the Prior Arts

After removal of a large area of a skull of a patient due to trauma or operation, cranioplasty needs to be carried out to repair the defect area in the skull using an artificial bone plate to cover the defect area of the skull. The artificial bone plate provides proper protection for the vulnerable brain tissue and prevents sequelae.

A conventional artificial bone plate used in cranioplasty is cut by machining from a chunk of material; to be precise, a chunk of metal or polymer is cut into the shape of the removed bone of the skull, and then the machined artificial bone plate is fixed to the skull by surgery.

However, the surface of the skull is curved, and therefore a great amount of material needs to be removed during the machining process, which causes a waste of material and time.

To overcome the shortcomings, the present invention provides an assembleable artificial bone plate and an artificial bone plate unit to mitigate or obviate the aforementioned problems.

The main objective of the present invention is to provide an assembleable artificial bone plate and artificial bone plate units that save material and take less time to manufacture.

An artificial bone plate unit of a first configuration comprises a plate body, multiple connecting pins and multiple connecting holes. The plate body has two main surfaces and a peripheral surface. The peripheral surface is connected between the two main surfaces. The connecting pins are formed on the plate body and arranged along the peripheral surface on the plate body. The connecting holes are formed in the plate body and arranged along the peripheral surface on the plate body. The connecting holes correspond in shape to the connecting pins. Multiple drug cavities are formed in the artificial bone plate unit. Multiple drug-releasing openings are formed on the artificial bone plate unit, and each of the drug-releasing openings is connected to a respective one of the drug cavities. A maximum area of a cross section of each of the drug cavities is greater than an area of a corresponding one of the drug-releasing openings; and a normal direction of the cross section of each of the drug cavities is parallel to an opening direction of the corresponding drug-releasing opening.

The assembleable artificial bone plate is bendable and comprises multiple aforementioned artificial bone plate units. The artificial bone plate units are connected using the connecting pins and the connecting holes.

By designing the artificial bone plate units each having the connecting pins and the connecting holes formed on/in the plate body and arranged along the peripheral surface, the artificial bone plate units can be connected with each other using the connecting pins and the connecting holes located in the edge to form a larger piece of an assembleable artificial bone plate. An artificial bone plate for the patient is produced by connecting multiple said artificial bone plate units of the present invention to form a larger piece of the assembleable artificial bone plate, and then bend the assembleable artificial bone plate to make its shape correspond to a shape of a defect area of a skull. In this case, a waste of expensive medical grade material due to machining is prevented, and also the time it takes to produce the artificial bone plate is shorter.

Moreover, with the drug cavities and the drug-releasing openings, the bone plate unit is capable of facilitating wound healing by slowly releasing drugs after a surgery. To be precise, the drugs can be filled in the drug cavities before the surgery, and then the drugs will be slowly released through the drug-releasing openings after the bone plate unit is placed inside a human body.

In addition, three USA patent publications, which are No. US 2022/354553 A1, No. US 2020/405353 A1, and No. US 2019/380836 A1, have respectively disclosed an artificial bone plate unit.

Other objectives, advantages and novel features of the invention will become more apparent from the following detailed description when taken in conjunction with the accompanying drawings.

**In the drawings:**
Fig. 1 is a perspective view of a first embodiment of an artificial bone plate unit in accordance with the present invention;
Fig. 2 is a top view of the artificial bone plate unit in Fig. 1;
Fig. 3 is an enlarged longitudinal section view of the artificial bone plate unit in Fig. 1;
Figs. 4 to 8 are enlarged longitudinal section views of other artificial bone plate units in accordance with the present invention;
Fig. 9 is a perspective view of a second embodiment of an artificial bone plate unit in accordance with the present invention;
Fig. 10 is a perspective view of a third embodiment of an artificial bone plate unit in accordance with the present invention;
Fig. 11 is a perspective view of a fourth embodiment of an artificial bone plate unit in accordance with the present invention;
Fig. 12 is a perspective view of a fifth embodiment of an artificial bone plate unit in accordance with the present invention;
Fig. 13 is a perspective view of a sixth embodiment of an artificial bone plate unit in accordance with the present invention;
Fig. 14 is a perspective view of a seventh embodiment of an artificial bone plate unit in accordance with the present invention;
Fig. 15 is an enlarged longitudinal section view of the artificial bone plate unit in Fig. 14;
Fig. 16 is a perspective view of a first embodiment of an assembleable artificial bone plate in accordance with the present invention;
Fig. 17 is a perspective view of an eighth embodiment of an artificial bone plate unit in accordance with the present invention;
Fig. 18 is another perspective view of the artificial bone plate unit in Fig. 17;
Fig. 19 is a perspective view of a ninth embodiment of an artificial bone plate unit in accordance with the present invention;
Fig. 20 is another perspective view of the artificial bone plate unit in Fig. 19; and
Fig. 21 is a perspective view of a second embodiment of an assembleable artificial bone plate in accordance with the present invention.

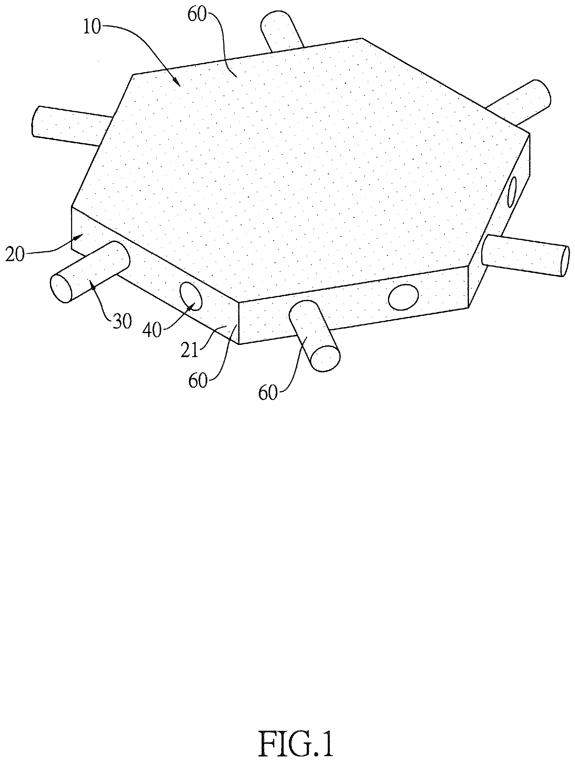

With reference to Figs. 1 to 3, an artificial bone plate unit in accordance with the present invention comprises a plate body, multiple connecting pins 30 multiple connecting holes 40, multiple drug cavities 50, and multiple drug-releasing openings 60. The plate body has two main surfaces 10, a peripheral surface 20, multiple connecting pins 30 and multiple connecting holes 40. The peripheral surface 20 is connected between the two main surfaces 10.

The connecting pins 30 and the connecting holes 40 are formed on the plate body and arranged along the peripheral surface 20 on the plate body. The connecting holes 40 correspond in shape to the connecting pins 30, which means two artificial bone plate units in accordance with the present invention can be assembled together by engaging one of the connecting pins 30 of one plate unit with one of the connecting holes 40 of the other plate unit.

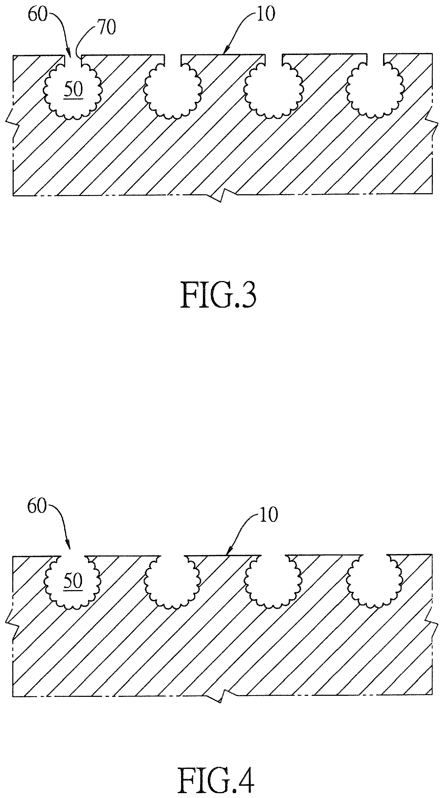

The drug cavities 50 (as shown in Fig. 3) are formed in the artificial bone plate unit. The drug-releasing openings 60 are formed on the artificial bone plate unit, and each of the drug-releasing openings 60 is connected to a respective one of the drug cavities 50 such that drugs can be prefilled in the drug cavities 50 before surgery, and then drugs in the drug cavities 50 will be slowly released after the artificial bone plate unit is placed inside a human body.

In the preferred embodiment, the drug cavities 50 are formed in the plate body and the connecting pins 30; the drug-releasing openings 60 are formed on the two main surfaces 10, the peripheral surface 20, and all outer surfaces of the connecting pins 30; that is, the drug-releasing openings 60 and the drug cavities 50 are disposed on all outer surfaces of the artificial bone plate unit. In another preferred embodiment, the drug-releasing openings 60 and the drug cavities 50 are disposed only on the plate body. The drug cavities 50 and the drug-releasing openings 60 are preferably formed together by etching.

Moreover, the artificial bone plate unit preferably has multiple drug-releasing channels 70. Each of the drug-releasing channels 70 connects a respective one of the drug-releasing openings 60 and a respective one of the drug cavities 50; that is, the drug-releasing openings 60 and the drug cavities 50 are connected via the drug-releasing channels 70. In another preferred embodiment, the drug-releasing channels 70 are omitted, and the drug-releasing openings 60 and the drug cavities 50 are directly connected.

With reference to Figs. 3 to 8, detailed dimensions and shapes of the drug cavities 50 are further explained using cross section and longitudinal section of the drug cavities 50. A normal direction of the cross section of each of the drug cavities 50 is parallel to an opening direction of the corresponding drug-releasing opening 60, while a normal direction of the longitudinal section of each of the drug cavities is perpendicular to an opening direction of the corresponding drug-releasing opening 60.

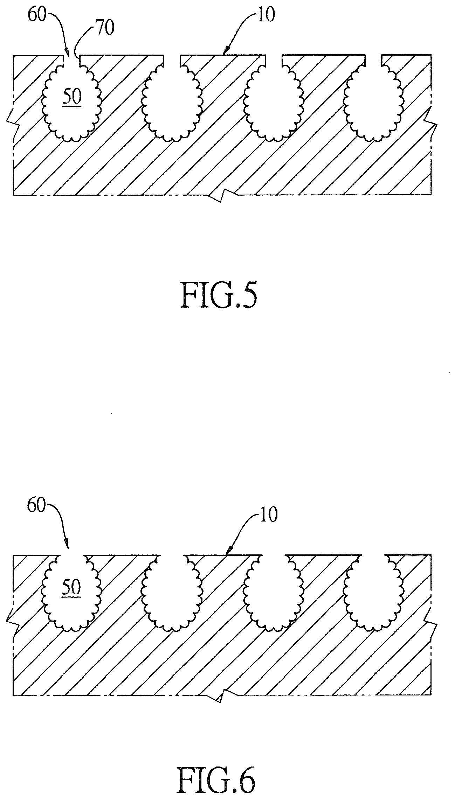

A maximum area of the cross section of each of the drug cavities 50 is greater than an area of a corresponding one of the drug-releasing openings 60. As a result, drug in the drug cavity 50 will be released slowly for a longer period of time due to the relatively small drug-releasing opening 60. Shape of a longitudinal section of each of the drug cavities 50 is a circle (as shown in Figs. 3 and 4), an ellipse (as shown in Figs. 5 and 6), or an ovoid.

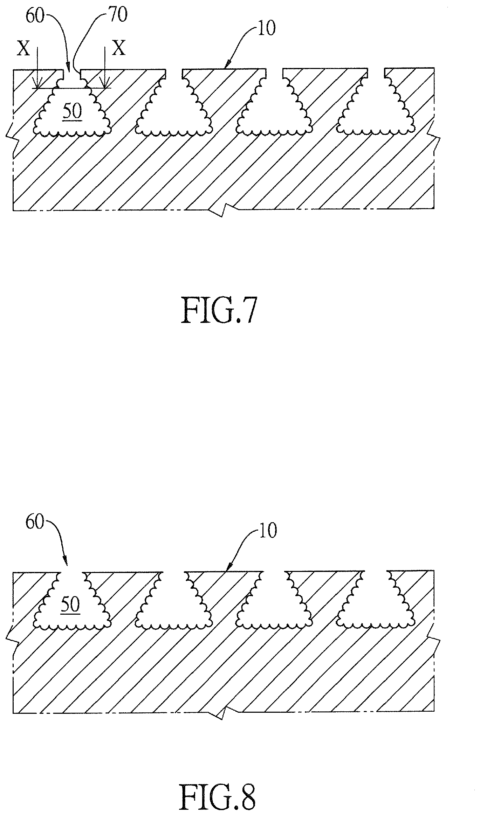

With reference to Figs. 7 and 8, in another preferred embodiment, the area of the cross section of each of the drug cavities 50 increases as a cutting plane X-X of the cross section moves away from the corresponding drug-releasing opening 60. In this particular embodiment, the shape of the longitudinal section of each of the drug cavities 50 is preferably a triangle.

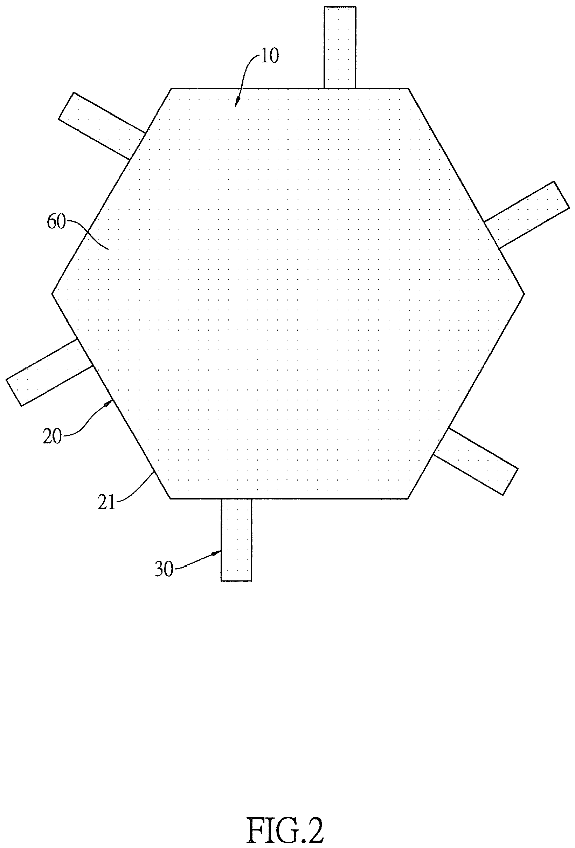

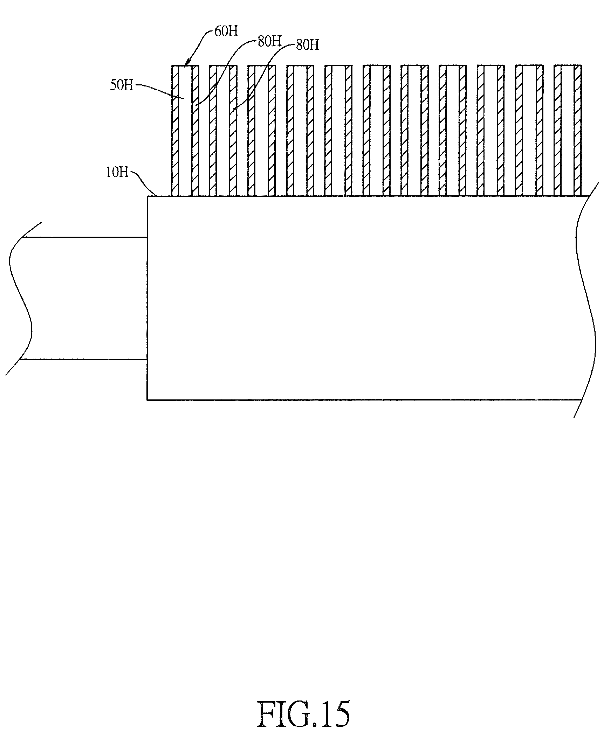

With reference to Figs. 1 and 2, the connecting pins 30 and the connecting holes 40 are preferably formed on the peripheral surface 20 of the plate body, and therefore when multiple said artificial bone plate units are assembled together to form an assembleable artificial bone plate, a surface of the assembleable artificial bone plate is substantially smooth as edges of the plate bodies do not protrude therefrom. However, positions of the connecting pins 30 and the connecting holes 40 are not limited by the abovementioned, as long as the connecting pins 30 and the connecting holes 40 are arranged along the peripheral surface 20 on/in the plate body, which means the connecting pins 30 and the connecting holes 40 only have to surround or be arranged along the peripheral surface 20, and do not have to be located on the peripheral surface 20. To be precise, the connecting pins 30 and the connecting holes 40 can be formed on the peripheral surface 20 as shown in Fig. 1, or the connecting pins 30F and the connecting holes 40F can be formed on the main surface 10F and located adjacent to an edge of the peripheral face 20F as shown in Fig.15.

The plate body in a preferred embodiment is a six-sided polygon, and to be precise, the plate body is a regular hexagon, such that the six connecting faces 21 are formed on the peripheral surface 20. A number of the connecting pins 30 is six, and each of the six connecting pins 30 is formed on a respective one of the six connecting faces. A number of the connecting holes 40 is six, and each of the six connecting holes 40 is formed in a respective one of the six connecting faces 21.

In another preferred embodiment, the plate body can be an N-sided polygon other than hexagon, where N is an integer greater than 2. When the plate body is the N-sided polygon other than hexagon, a number of the connecting faces 21, the number of the connecting pins 30, and the number of the connecting holes 40 are N. Each of the N connecting pins 30 is formed on a respective one of the N connecting faces 21, and each of the N connecting holes 40 is formed in a respective one of the N connecting faces 21. In other words, each side of the N-sided polygonal plate body has a connecting pin 30 and a connecting hole 40.

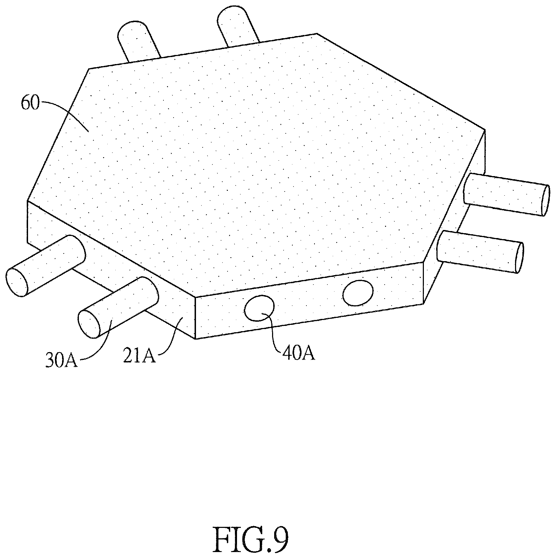

Moreover, a number of the connecting pin 30 on each connecting face 21 and a number of the connecting hole 40 on each connecting face 21 are not limited to one. For example, in a second embodiment in accordance with the present invention (as shown in Fig. 9), some connecting faces 21A have two connecting pins 30A but no connecting hole 40A, while other connecting faces 21A have two connecting holes 40A but no connecting pins 30A.

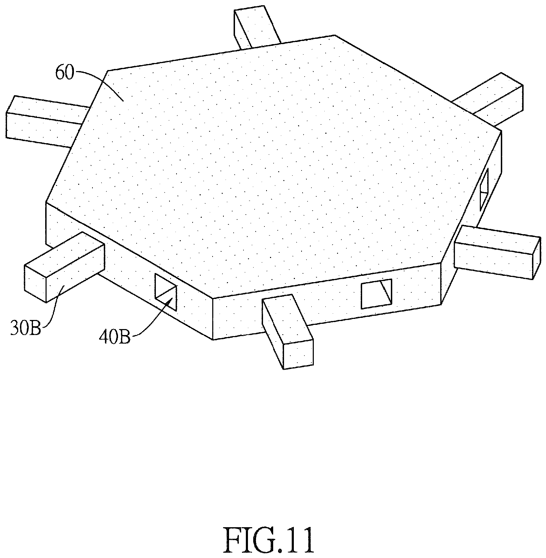

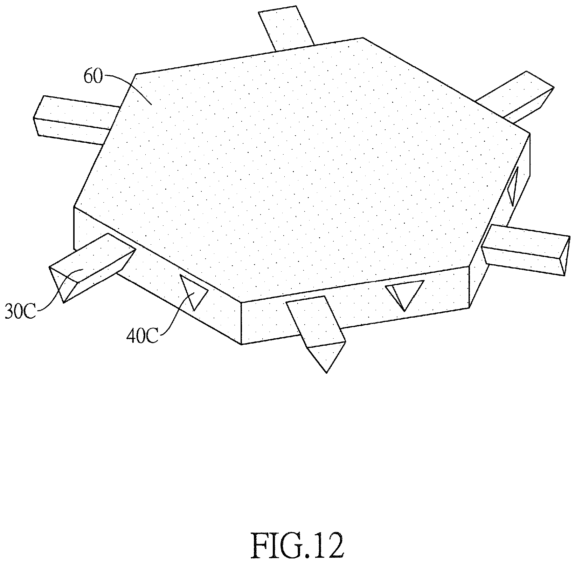

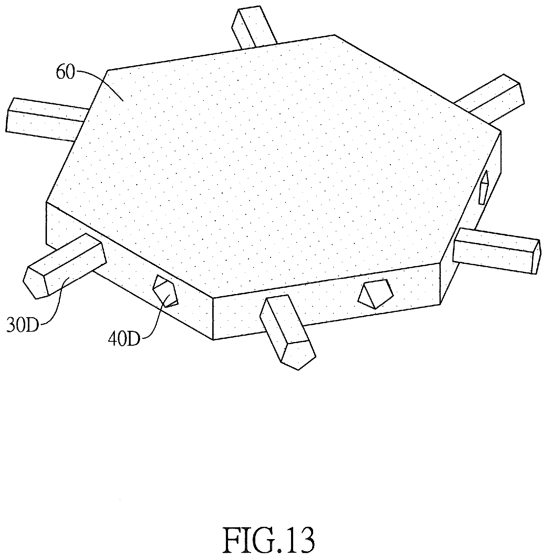

A shape of the connecting pin 30 is cylindrical, while the connecting hole 40 is a round recess, which corresponds to the shape of the connecting pin 30. However, the shapes of the connecting pin 30 and connecting hole 40 are not limited to the abovementioned, and can be of other shapes depending on the application. For example, the connecting pin 30B can be a quadrilateral prism as shown in Fig 11, while the connecting hole 40B is a square hole; or the connecting pin 30C can be a triangular prism as shown in Fig 12, while the connecting hole 40C is a triangular hole; or the connecting pin 30D can be a pentagonal prism as shown in Fig 13, while the connecting hole 40D is a pentagonal hole.

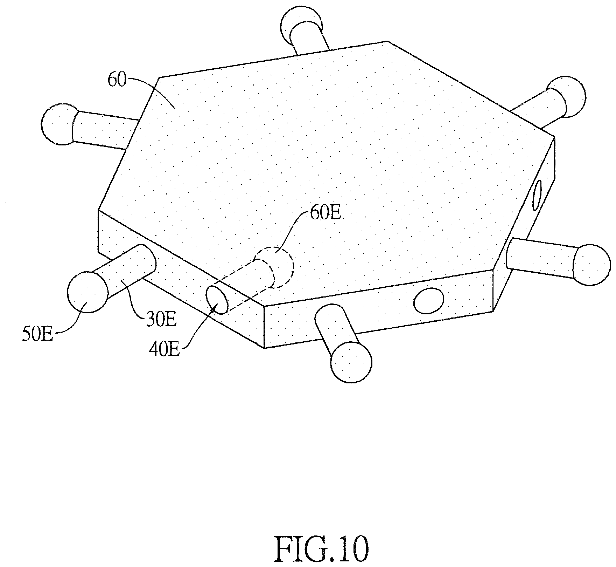

In another preferred embodiment, a round connecting ball 50E is connected to the tip of the connecting pin 30E (as shown in Fig. 10), and a diameter of the connecting ball 50E is greater than a diameter of the connecting pin 30E. A round connecting cavity 60E is formed in a bottom of the round connecting hole 40E, and a diameter of the connecting cavity 60E corresponds to the diameter of the connecting ball 50E. In this case, when connecting two of the artificial bone plate units, the connecting ball 50E can be pressed through the connecting hole 40E and forcing a diameter of the connecting hole 40E to expand elastically, and finally the connecting ball 50E is pressed through the connecting hole 40E and engages with the connecting cavity 60E. A strength of the connection between said two artificial bone plate units is further enhanced to prevent separation when the two artificial bone plate units are pulled in opposite directions respectively.

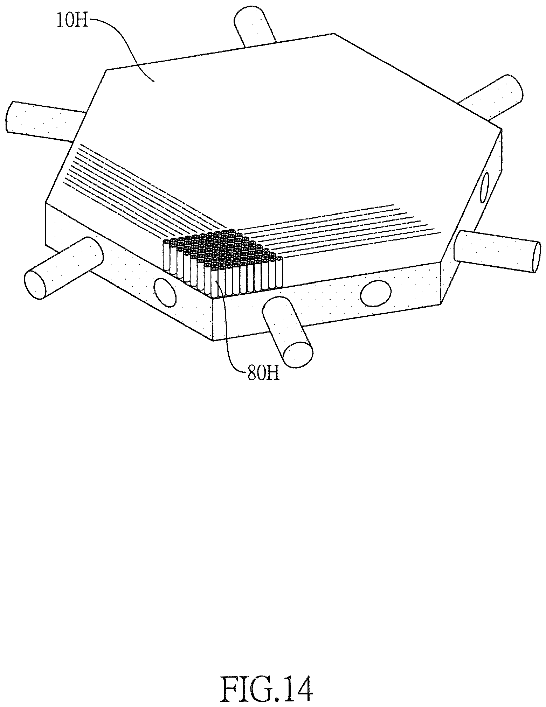

With reference to Figs. 14 and 15, a seventh embodiment of an artificial bone plate unit in accordance with the present invention is substantially similar to the first embodiment, but the difference is that multiple drug pins 80H are formed on one of the main surfaces 10H of the plate body. Each of the drug pins 80H has one of the drug cavities 50H formed in the drug pin and one of the drug-releasing openings 60H formed on a distal end of the drug pin 80H and connected to the corresponding drug cavity 50H. The drug pins 80H are preferably micro tubes formed by special chemical procedures, and are preferably intensely arranged the main surface 10H.

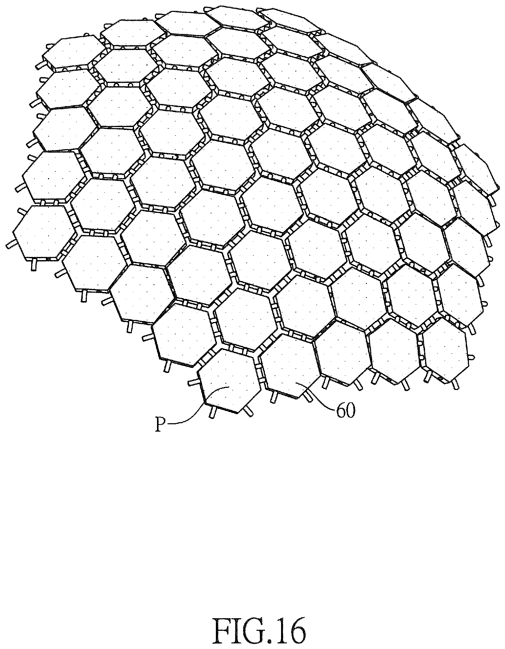

With reference to Fig. 16, an assembleable artificial bone plate in accordance with the present invention is bendable. The assembleable artificial bone plate comprises multiple said artificial bone plate units P. The artificial bone plate units P are connected to each other. Among any two of the artificial bone plate units P that are connected to each other, at least one of the connecting pins 30 on one of said two artificial bone plate units P is mounted inside the at least one of the connecting holes 40 in the other one of said two artificial bone plate units P. In a preferred embodiment, the assembleable artificial bone plate is formed by having the artificial bone plate units P connected to each other through the connecting pins 30 and the connecting holes 40 located on the peripheral surface 20, and the artificial bone plate units P are in accordance with the first embodiment of the present invention, but an assembleable artificial bone plate can also be formed by the artificial bone plate units of another embodiment.

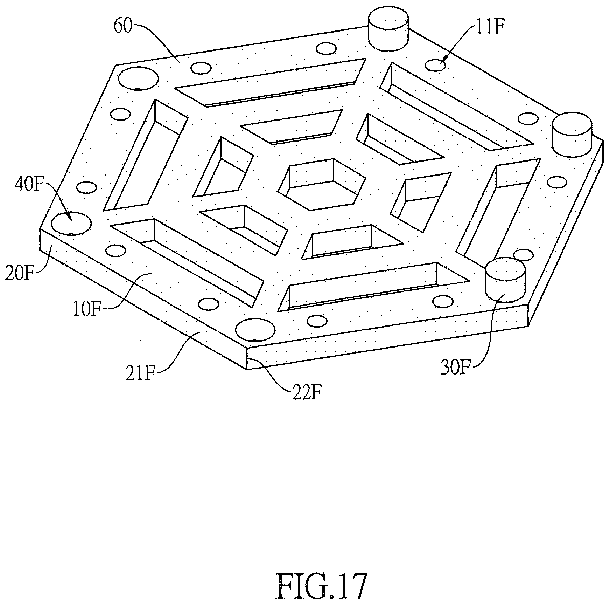

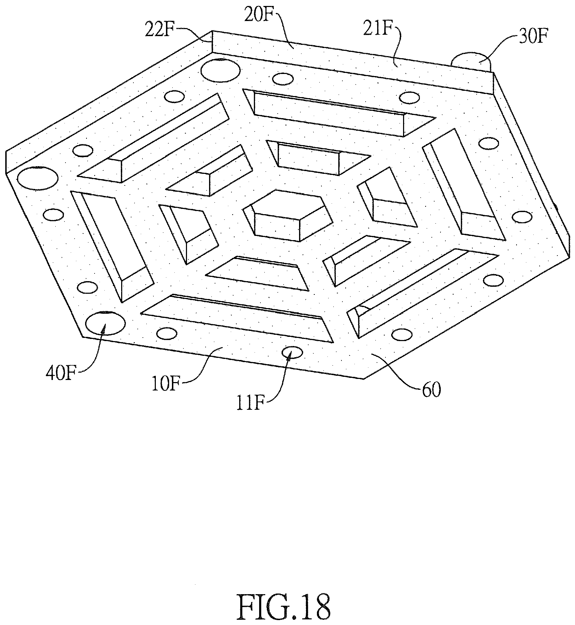

With reference to Figs. 17 and 18, an eighth embodiment of the artificial bone plate unit in accordance with the present invention is substantially similar to the first embodiment mentioned above, but the connecting pins 30F and the connecting holes 40F are formed in different positions.

The connecting pins 30F are formed on one of the two main surfaces 10F. The connecting holes 40F are formed through the two main surfaces 10F of the artificial bone plate unit. In a preferred embodiment, multiple screw holes 11F are formed through the two main surfaces 10F, and the screw holes 11F are arranged along the peripheral face 20F of the plate body. However, positions of the screw holes 11F are not limited by abovementioned positions, and the plate body may optionally have no screw holes 11F.

In a preferred embodiment, the plate body is polygonal, such that multiple corner portions 22F are formed on the peripheral surface 20F. Each of the connecting pins 30F is disposed adjacent to one of the corner portions 22F, and each of the connecting holes 40F is disposed adjacent to one of the corner portions 22F. To be precise, the plate body is a six-sided polygon, and the corner portions 22F are located adjacent to the corner positions of the polygonal plate body. However, positions of the connecting pins 30F and the connecting holes 40F are not limited by abovementioned positions. For example, the connecting pins 30F and the connecting holes 40F can be located in the middle of two of the adjacent corner portions 22F.

In a preferred embodiment, the plate body is an N-sided polygon, such that the peripheral surface 20F comprises N connecting faces 21F. A sum of a number of the connecting pins 30F and a number of the connecting holes 40F is N. To be precise, N is six, and the plate body is hexagonal, such that the peripheral surface 20F comprises N connecting faces 21F. Three connecting pins 30F and three connecting holes 40F are formed on the plate body, making the sum of the number of the connecting pins 30F and the number of the connecting holes 40F be six, through which a polygonal plate body can be securely connected to an adjacent polygonal plate body. However, the sum of the number of the connecting pins 30F and the number of the connecting holes 40F is not limited by the abovementioned. For example, the sum of the number of the connecting pins 30F and the number of the connecting holes 40F in the polygonal plate body can be twelve.

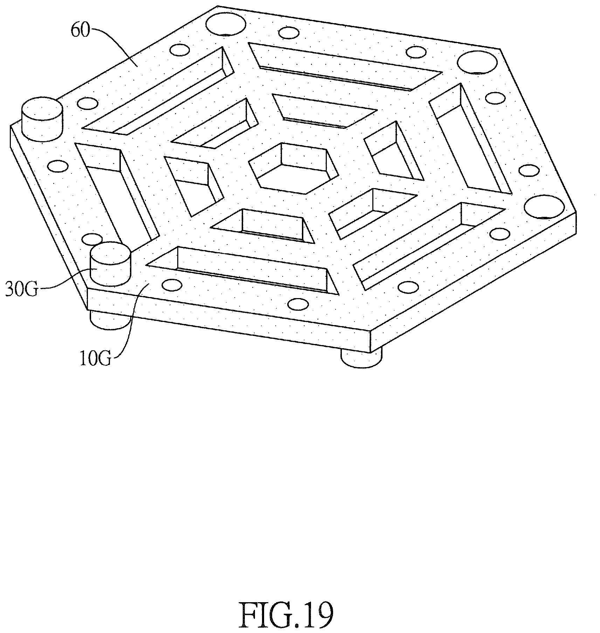

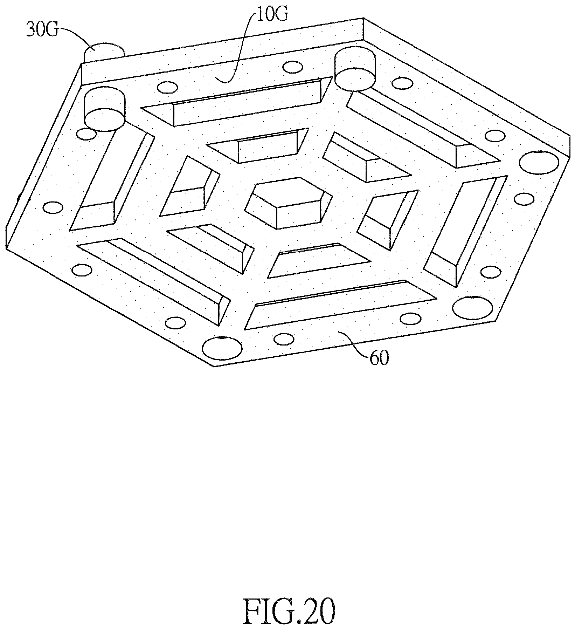

With reference to Figs. 19 and 20, an ninth embodiment of the artificial bone plate unit is substantially similar to the eighth embodiment mentioned above, but the connecting pins 30G are formed on a respective one of the two main surfaces 10G. The connecting pins 30G can be located symmetrically on the two main surfaces 10G, which means all connecting pins 30G are formed through the plate body; the connecting pins 30G can also be located asymmetrically, which means some of the connecting pins 30G are formed through the plate body, while the remaining connecting pins 30G are only formed on either side of the plate body.

A distance between the two main surfaces 10F is defined as a thickness of the plate body. The thickness of the plate body in the aforementioned eighth and ninth embodiments of the artificial bone plate unit is less than 1mm, but the thickness of the plate body in the embodiment wherein the connecting pins 30F and the connecting holes 40F are formed on the main surface 10F is not limited by abovementioned as long as the plate body can be deformed when bended to satisfy the need of a surgical repair.

In all the abovementioned embodiments of the artificial bone plate unit, the artificial bone plate unit can be made of a material with good biocompatibility and mechanical strength, such as titanium, Ti-6Al-4V, 316L stainless steel or polyether ether ketone (PEEK). The artificial bone plate unit can also be made of aluminum for reduced weight and cost. Moreover, all the abovementioned embodiments of the artificial bone plate unit comprise the drug cavities 50 and the drug-releasing openings 60 for releasing the drugs.

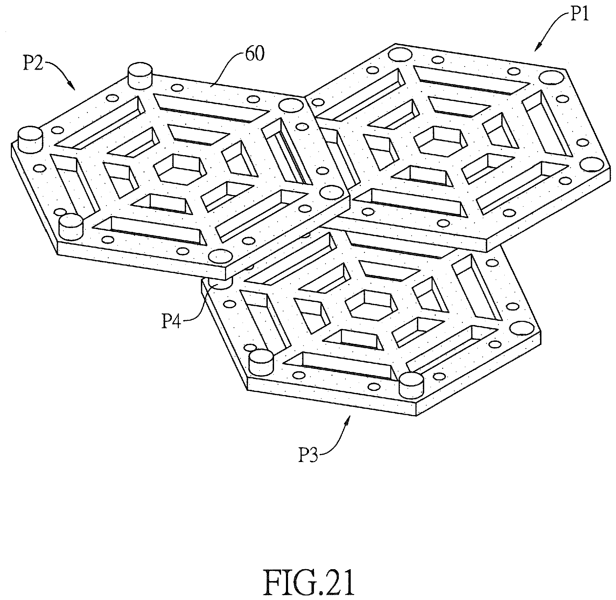

With reference to Fig. 21, a second embodiment of the assembleable artificial bone plate is substantially similar to the first embodiment mentioned above, but the difference is that the connecting pins and the connecting holes are formed on the main surfaces of the artificial bone plate units, and therefore the edges of the artificial bone plate units are overlapped. Three different embodiments of the artificial bone plate units are present in said assembleable artificial bone plate: the artificial bone plate unit P1 corresponds to the ninth embodiment as shown in Fig. 19; the artificial bone plate unit P2 corresponds to the eighth embodiment as shown in Fig. 17; the artificial bone plate unit P3 is substantially similar to the eighth embodiment mentioned above, but a length of one of the connecting pins P4 is longer than the other connecting pins in order to connect the artificial bone plate unit P2.

To use the present invention, first connect several artificial bone plate units together to form a larger piece of the assembleable artificial bone plate, and then bend the assembleable artificial bone plate to make the shape of the assembleable artificial bone plate correspond to the shape of a defect area of a skull.

When the assembleable artificial bone plate is of the second embodiment (as shown Fig. 21), the edge of the assembleable artificial bone plate overlaps with the surface of the skull, and screws can be used to fasten the assembleable artificial bone plate to the skull through the screw holes on the artificial bone plate units.

When the assembleable artificial bone plate is of the first embodiment (as shown Fig. 16), the assembleable artificial bone plate can be embedded and fixed to the defect area of the skull after trimming the edge of the assembleable artificial bone plate properly.

## Claims

1. An artificial bone plate unit (P) comprising:
a plate body having
two main surfaces (10);
a peripheral surface (20) connected between the two main surfaces (10);
multiple connecting pins (30) formed on the plate body and arranged along the peripheral surface (20) on the plate body;
multiple connecting holes (40) formed in the plate body and arranged along the peripheral surface (20) in the plate body; the connecting holes (40) corresponding in shape to the connecting pins (30);
**characterized in that**
multiple drug cavities (50) are formed in the artificial bone plate unit (P); and
multiple drug-releasing openings (60) are formed on the artificial bone plate unit (P); each of the drug-releasing openings (60) is connected to a respective one of the drug cavities (50);
a maximum area of a cross section of each of the drug cavities (50) is greater than an area of a corresponding one of the drug-releasing openings (60); and
a normal direction of the cross section of each of the drug cavities (50) is parallel to an opening direction of the corresponding drug-releasing opening (60).

2. The artificial bone plate unit (P) as claimed in claim 1, comprising multiple drug-releasing channels (70); each of the drug-releasing channels (70) connecting a respective one of the drug-releasing openings (60) and a respective one of the drug cavities (50).

3. The artificial bone plate unit (P) as claimed in claim 1, wherein:
a shape of a longitudinal section of each of the drug cavities (50) is a circle, an ellipse, an ovoid, or a triangle; and
a normal direction of the longitudinal section of each of the drug cavities (50) is perpendicular to the opening direction of the corresponding drug-releasing opening (60).

4. The artificial bone plate unit (P) as claimed in claim 1, wherein:
an area of the cross section of each of the drug cavities (50) increases as a cutting plane of the cross section moves away from the corresponding drug-releasing opening (60).

5. The artificial bone plate unit (P) as claimed in any of claims 1 to 4, wherein the connecting pins (30) and the connecting holes (40) are located on the peripheral surface (20) of the plate body.

6. The artificial bone plate unit (P) as claimed in claim 5, wherein
the plate body is an N-sided polygon such that the peripheral surface (20) comprises N connecting faces (21);
a number of the connecting pins (30) is N, and each of the connecting pins (30) is formed on a respective one of the N connecting faces (21);
a number of the connecting holes (40) is N, and each of the connecting holes (40) is formed in a respective one of the N connecting faces (21);
wherein N is an integer greater than two.

7. The artificial bone plate unit (P) as claimed in any of claims 1 to 4, wherein
the connecting pins (30) are formed on one of the two main surfaces (10);
the connecting holes (40) are formed through the two main surfaces (10).

8. The artificial bone plate unit (P) as claimed in any of claims 1 to 4, wherein
the connecting pins (30) are formed on the two main surfaces (10);
the connecting holes (40) are formed through the two main surfaces (10).

9. The artificial bone plate unit (P) as claimed in claim 7, wherein multiple screw holes (11F) are formed through the two main surfaces (10F) and are arranged along the peripheral surface (20F).

10. The artificial bone plate unit (P) as claimed in any of claims 1 to 4, wherein the plate body is polygonal, such that the peripheral surface (20F) comprises multiple corner portions (22F); each of the connecting pins (30F) is disposed adjacent to one of the corner portions (22F); each of the connecting holes (40F) is disposed adjacent to one of the corner portions (22F).

11. The artificial bone plate unit (P) as claimed in any of claims 1 to 4, wherein the plate body is an N-sided polygon, such that the peripheral surface (20) comprises N connecting faces (21); a sum of a number of the connecting pins (30) and a number of the connecting holes (40) is N; N is an integer greater than 2.

12. The artificial bone plate unit (P) as claimed in claim 7, wherein a thickness of the plate body is less than 1mm.

13. The artificial bone plate unit (P) as claimed in any of claims 1 to 4, wherein the artificial bone plate unit (P) is made of titanium, Ti-6Al-4V, 316L stainless steel, polyether ether ketone, or aluminum.

14. The artificial bone plate unit (P) as claimed in any of claims 1 to 4, wherein multiple drug pins (80H) formed on one of the main surfaces (10H) of the plate body; each of the drug pins (80H) has
one of the drug cavities (50H) formed in the drug pin (80H);
one of the drug-releasing openings (60H) formed on a distal end of the drug pin (80H) and connected to the corresponding drug cavity.

15. An assembleable artificial bone plate, **characterized in that** the assembleable artificial bone plate is bendable and comprises multiple said artificial bone plate units (P) as claimed in any of claims 1 to 14; the artificial bone plate units (P) are connected to each other; among any two of the artificial bone plate units (P) that are connected to each other, at least one of the connecting pins (30) on one of said two artificial bone plate units (P) are mounted inside a respective one of the connecting holes (40) in the other one of said two artificial bone plate units (P).

## Patentansprüche

1. Künstliche Knochenplatteneinheit (P), die Folgendes umfasst:
einen Plattenkörper, der Folgendes aufweist:
zwei Hauptflächen (10);
eine Umfangsfläche (20), die zwischen den zwei Hauptflächen (10) verbunden ist;
mehrere Verbindungsstifte (30), die an dem Plattenkörper geformt und entlang der Umfangsfläche (20) an dem Plattenkörper angeordnet sind;
mehrere Verbindungslöcher (40), die in dem Plattenkörper geformt und entlang der Umfangsfläche (20) in dem Plattenkörper angeordnet sind, wobei die Verbindungslöcher (40) in der Form den Verbindungsstiften (30) entsprechen;
**dadurch gekennzeichnet, dass**
mehrere Arzneimittel-Hohlräume (50) in der künstlichen Knochenplatteneinheit (P) geformt sind; und
mehrere Arzneimittel-Freisetzungsöffnungen (60) an der künstlichen Knochenplatteneinheit (P) geformt sind; wobei jede von den Arzneimittel-Freisetzungsöffnungen (60) mit einem jeweiligen von den Arzneimittel-Hohlräumen (50) verbunden ist;
eine maximale Oberfläche eines Querschnitts jedes von den Arzneimittel-Hohlräumen (50) größer ist als eine Oberfläche einer entsprechenden von den Arzneimittel-Freisetzungsöffnungen (60); und
eine Normalrichtung des Querschnitts jedes von den Arzneimittel-Hohlräumen (50) parallel zu einer Öffnungsrichtung der entsprechenden Arzneimittel-Freisetzungsöffnung (60) ist.

2. Künstliche Knochenplatteneinheit (P) nach Anspruch 1, die mehrere Arzneimittel-Freisetzungskanäle (70) umfasst; wobei jeder von den Arzneimittel-Freisetzungskanälen (70) eine jeweilige von den Arzneimittel-Freisetzungsöffnungen (60) und einen jeweiligen von den Arzneimittel-Hohlräumen (50) verbindet.

3. Künstliche Knochenplatteneinheit (P) nach Anspruch 1, wobei:
eine Form eines Längsschnitts jedes von den Arzneimittel-Hohlräumen (50) ein Kreis, eine Ellipse, ein Oval oder ein Dreieck ist; und
eine Normalrichtung des Längsschnitts jedes von den Arzneimittel-Hohlräumen (50) senkrecht zu der Öffnungsrichtung der entsprechenden Arzneimittel-Freisetzungsöffnung (60) ist.

4. Künstliche Knochenplatteneinheit (P) nach Anspruch 1, wobei:
eine Oberfläche des Querschnitts jedes von den Arzneimittel-Hohlräumen (50) zunimmt, wenn sich eine Schnittebene des Querschnitts von der entsprechenden Arzneimittel-Freisetzungsöffnung (60) weg bewegt.

5. Künstliche Knochenplatteneinheit (P) nach einem der Ansprüche 1 bis 4, wobei sich die Verbindungsstifte (30) und die Verbindungslöcher (40) an der Umfangsfläche (20) des Plattenkörpers befinden.

6. Künstliche Knochenplatteneinheit (P) nach Anspruch 5, wobei
der Plattenkörper ein N-seitiges Vieleck ist, so dass die Umfangsfläche (20) N Verbindungsflächen (21) umfasst;
eine Anzahl der Verbindungsstifte (30) N ist und jeder von den Verbindungsstiften (30) an einer jeweiligen der N Verbindungsflächen (21) geformt ist;
eine Anzahl der Verbindungslöcher (40) N ist und jedes von den Verbindungslöchern (40) in einer jeweiligen der N Verbindungsflächen (21) geformt ist;
wobei N eine ganze Zahl, größer als zwei, ist.

7. Künstliche Knochenplatteneinheit (P) nach einem der Ansprüche 1 bis 4, wobei
die Verbindungsstifte (30) an einer von den zwei Hauptflächen (10) geformt sind;
die Verbindungslöcher (40) durch die zwei Hauptflächen (10) geformt sind.

8. Künstliche Knochenplatteneinheit (P) nach einem der Ansprüche 1 bis 4, wobei
die Verbindungsstifte (30) an den zwei Hauptflächen (10) geformt sind;
die Verbindungslöcher (40) durch die zwei Hauptflächen (10) geformt sind.

9. Künstliche Knochenplatteneinheit (P) nach Anspruch 7, wobei mehrere Schraubenlöcher (11F) durch die zwei Hauptflächen (10F) geformt sind und entlang der Umfangsfläche (20F) angeordnet sind.

10. Künstliche Knochenplatteneinheit (P) nach einem der Ansprüche 1 bis 4, wobei der Plattenkörper vieleckig ist, so dass die Umfangsfläche (20F) mehrere Eckabschnitte (22F) umfasst; wobei jeder von den Verbindungsstiften (30F) angrenzend an einen von den Eckabschnitten (22F) angeordnet ist; wobei jedes von den Verbindungslöchern (40F) angrenzend an einen von den Eckabschnitten (22F) angeordnet ist.

11. Künstliche Knochenplatteneinheit (P) nach einem der Ansprüche 1 bis 4, wobei der Plattenkörper ein N-seitiges Vieleck ist, so dass die Umfangsfläche (20) N Verbindungsflächen (21) umfasst; wobei eine Summe einer Anzahl der Verbindungsstifte (30) und einer Anzahl der Verbindungslöcher (40) N ist; wobei N eine ganze Zahl, größer als zwei, ist.

12. Künstliche Knochenplatteneinheit (P) nach Anspruch 7, wobei eine Dicke des Plattenkörpers weniger als 1 mm beträgt.

13. Künstliche Knochenplatteneinheit (P) nach einem der Ansprüche 1 bis 4, wobei die künstliche Knochenplatteneinheit (P) aus Titan, Ti-6Al-4V, rostfreiem Stahl 316L, Polyetheretherketon oder Aluminium hergestellt ist.

14. Künstliche Knochenplatteneinheit (P) nach einem der Ansprüche 1 bis 4, wobei mehrere Arzneimittelstifte (80H) an einer der Hauptflächen (10H) des Plattenkörpers geformt sind; wobei jeder von den Arzneimittelstiften (80H) Folgendes aufweist
einen von den Arzneimittel-Hohlräumen (50H), der in dem Arzneimittelstift (80H) geformt ist;
eine von den Arzneimittel-Freisetzungsöffnungen (60H), die an einem distalen Ende des Arzneimittelstifts (80H) geformt und mit dem entsprechenden Arzneimittel-Hohlraum verbunden ist.

15. Zusammenbaubare künstliche Knochenplatte, **dadurch gekennzeichnet, dass** die zusammenbaubare künstliche Knochenplatte biegsam ist und mehrere künstliche Knochenplatteneinheiten (P) nach einem der Ansprüche 1 bis 14 umfasst; wobei die künstlichen Knochenplatteneinheiten (P) miteinander verbunden sind; wobei unter beliebigen zwei von den künstlichen Knochenplatteneinheiten (P), die miteinander verbunden sind, mindestens einer von den Verbindungsstiften (30) an der einen von den zwei künstlichen Knochenplatteneinheiten (P) innerhalb eines jeweiligen von den Verbindungslöchern (40) in der anderen von den zwei künstlichen Knochenplatteneinheiten (P) angebracht ist.

## Revendications

1. Unité de plaque osseuse artificielle (P) comprenant :
un corps de plaque présentant
deux surfaces principales (10) ;
une surface périphérique (20) située entre les deux surfaces principales (10) ;
plusieurs tiges de liaison (30) formées sur le corps de plaque et agencées le long de la surface périphérique (20) sur le corps de plaque ;
plusieurs trous de liaison (40) formés dans le corps de plaque et agencés le long de la surface périphérique (20) dans le corps de plaque ; les trous de liaison (40) correspondant de par leur forme aux tiges de liaison (30) ;
**caractérisée en ce que**
plusieurs cavités de médicament (50) sont formées dans l'unité de plaque osseuse artificielle (P) ; et
plusieurs ouvertures de libération de médicament (60) sont formées sur l'unité de plaque osseuse artificielle (P) ; chacune des ouvertures de libération de médicament (60) est reliée à une cavité respective des cavités de médicament (50) ;
une surface maximale d'une section transversale de chacune des cavités de médicament (50) est supérieure à une surface d'une ouverture correspondante des ouvertures de libération de médicament (60) ; et
une direction normale de la section transversale de chacune des cavités de médicament (50) est parallèle à une direction d'ouverture de l'ouverture de libération de médicament (60) correspondante.

2. Unité de plaque osseuse artificielle (P) selon la revendication 1, comprenant plusieurs canaux de libération de médicament (70) ; chacun des canaux de libération de médicament (70) reliant une ouverture respective des ouvertures de libération de médicament (60) et une cavité respective des cavités de médicament (50).

3. Unité de plaque osseuse artificielle (P) selon la revendication 1, dans laquelle :
une forme d'une section longitudinale de chacune des cavités de médicament (50) est un cercle, une ellipse, un ovoïde ou un triangle ; et
une direction normale de la section longitudinale de chacune des cavités de médicament (50) est perpendiculaire à la direction d'ouverture de l'ouverture de libération de médicament (60) correspondante.

4. Unité de plaque osseuse artificielle (P) selon la revendication 1, dans laquelle :
une surface de la section transversale de chacune des cavités de médicament (50) augmente à mesure qu'un plan de coupe de la section transversale s'éloigne de l'ouverture de libération de médicament (60) correspondante.

5. Unité de plaque osseuse artificielle (P) selon l'une quelconque des revendications 1 à 4, dans laquelle les tiges de liaison (30) et les trous de liaison (40) sont situés sur la surface périphérique (20) du corps de plaque.

6. Unité de plaque osseuse artificielle (P) selon la revendication 5, dans laquelle
le corps de plaque est un polygone à N côtés de sorte que la surface périphérique (20) comprend N faces de liaison (21) ;
les tiges de liaison (30) sont au nombre de N, et chacune des tiges de liaison (30) est formée sur une face respective des N faces de liaison (21) ;
les trous de liaison (40) sont au nombre de N, et chacun des trous de liaison (40) est formé dans une face respective des N faces de liaison (21) ;
dans laquelle N est un nombre entier supérieur à deux.

7. Unité de plaque osseuse artificielle (P) selon l'une quelconque des revendications 1 à 4, dans laquelle
les tiges de liaison (30) sont formées sur l'une des deux surfaces principales (10) ;
les trous de liaison (40) sont formés à travers les deux surfaces principales (10).

8. Unité de plaque osseuse artificielle (P) selon l'une quelconque des revendications 1 à 4, dans laquelle
les tiges de liaison (30) sont formées sur les deux surfaces principales (10) ;
les trous de liaison (40) sont formés à travers les deux surfaces principales (10).

9. Unité de plaque osseuse artificielle (P) selon la revendication 7, dans laquelle plusieurs trous de vis (11F) sont formés à travers les deux surfaces principales (10F) et sont agencés le long de la surface périphérique (20F).

10. Unité de plaque osseuse artificielle (P) selon l'une quelconque des revendications 1 à 4, dans laquelle le corps de plaque est polygonal, de sorte que la surface périphérique (20F) comprend plusieurs parties d'angle (22F) ; chacune des tiges de liaison (30F) est disposée de manière adjacente à l'une des parties d'angle (22F) ; chacun des trous de liaison (40F) est disposé de manière adjacente à l'une des parties d'angle (22F).

11. Unité de plaque osseuse artificielle (P) selon l'une quelconque des revendications 1 à 4, dans laquelle le corps de plaque est un polygone à N côtés, de sorte que la surface périphérique (20) comprend N faces de liaison (21) ; une somme d'un nombre des tiges de liaison (30) et d'un nombre des trous de liaison (40) est N ; N est un nombre entier supérieur à 2.

12. Unité de plaque osseuse artificielle (P) selon la revendication 7, dans laquelle une épaisseur du corps de plaque est inférieure à 1 mm.

13. Unité de plaque osseuse artificielle (P) selon l'une quelconque des revendications 1 à 4, dans laquelle l'unité de plaque osseuse artificielle (P) est en titane, Ti-6A1-4V, acier inoxydable 316L, polyétheréthercétone ou aluminium.

14. Unité de plaque osseuse artificielle (P) selon l'une quelconque des revendications 1 à 4, dans laquelle plusieurs tiges de médicament (80H) sont formées sur l'une des surfaces principales (10H) du corps de plaque ; chacune des tiges de médicament (80H) présente
l'une des cavités de médicament (50H) formées dans la tige de médicament (80H) ;
l'une des ouvertures de libération de médicament (60H) formée sur une extrémité distale de la tige de médicament (80H) et reliée à la cavité de médicament correspondante.

15. Plaque osseuse artificielle assemblable, **caractérisée en ce que** la plaque osseuse artificielle assemblable est pliable et comprend plusieurs dites unités de plaque osseuse artificielle (P) selon l'une quelconque des revendications 1 à 14 ; les unités de plaque osseuse artificielle (P) sont reliées entre elles ; parmi une quelconque paire des unités de plaque osseuse artificielle (P) qui sont reliées entre elles, au moins l'une des tiges de liaison (30) sur l'une de ladite paire d'unités de plaque osseuse artificielle (P) est montée à l'intérieur d'un trou respectif des trous de liaison (40) dans l'autre de ladite paire d'unités de plaque osseuse artificielle (P) .
